# EUROPEAN PATENT APPLICATION

(11) **EP 1 985 191 A1**
(43) Date of publication of application: **29.10.2008**
(21) Application number: 07008593.1
(22) Date of filing: 27.04.2007
(51) Int. Cl.: A23L 1/30, A61K 31/216, A61K 35/74

(54) **Preparation of polyfunctional fermented food products**

(71) Applicant: UNIVERSITA' DEGLI STUDI DI MILANO, 20122 Milano (IT)
(72) Inventor: Guglielmetti, Simone Domenico, 20134 Milano (IT); Mora, Diego, 20133 Milano (IT)
(74) Representative: Minoja, Fabrizio

(57) **Abstract**

The present invention relates to an antioxidant fermented food product obtainable by fermenting a food matrix with a high content of hydroxycinnamic acid derivatives, such as chlorogenic acid, in the presence of a microorganism of the *Lactobacillus* species having cinnamoyl esterase activity; in particular, the invention relates to a food product obtained from apple pulp and/or juice, milk, green tea and ascorbic acid.

## Description

### Field of the invention

The present invention relates to polyfunctional fermented food products, in particular to fermented food products having a high content of free caffeic acid.

### State of the art

Hydroxycinnamic acids such as caffeic, ferulic, sinapic and *p*-coumaric acids are found in almost every plant (Herrmann, 1976; Kuhnau, 1976) and represent a major class of phenolic compounds. The most abundant hydroxycinnamic acid is caffeic acid (I) 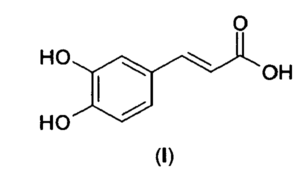 which is contained in foods mainly as ester with quinic acid, called chlorogenic acid (5-caffeoylquinic acid) (II)

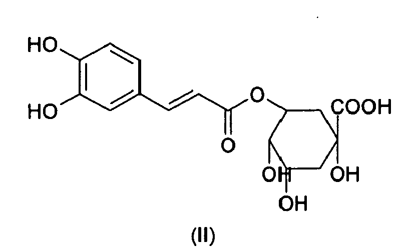

The main dietary sources of chlorogenic acid are coffee, tea, apples, pears, berries, artichokes and aubergines (Clifford, 1999).

Caffeic acid and chlorogenic acid have been demonstrated to possess remarkably high antioxidant activity *in vitro* and they have been associated with several health promoting effects *in vivo.* Particularly, they scavenge radicals generated in aqueous phase (Rice-Evans et al., 1996; Foley et al., 1999), enhance the *ex vivo* resistance of lipoproteins to oxidation (Nardini et al., 1995), inhibit DNA damage (Shibata et al., 1999; Kasai et al., 2000) and they are inhibitors of N-nitrosation reactions that might promote the formation of carcinogenic compounds (Kono et al., 1995). Moreover, *in vivo* studies carried out on humans and animals reported that caffeic and chlorogenic acids might prevent different cancers and cardiovascular diseases (Mori et al., 1986; Tanaka et al., 1990, 1993; Zhou et al., 1993; Suzuki et al., 2002) and increase the plasma antioxidant power and the concentration of endogenous antioxidants such as vitamin E (Tsuchiya et al., 1996; Natella et al., 2002; Lafay et al., 2005). Furthermore, caffeic acid has been associated to an antidepressive-like effect in mice (Takeda et al., 2002).

The biological properties of hydroxycinnamic acids depend on their absorption in the gastrointestinal tract. It has been demonstrated that the absorption of caffeic acid is drastically reduced when this molecule is ingested as chlorogenic acid (Spencer et al., 1999; Olthof et al., 2001; Gonthier et al., 2003; Lafay et al., 2006). Chlorogenic acid reaches the large intestine where it is quickly hydrolyzed by bacteria having cinnamoyl esterase activity and is extensively further degraded by the microbiota (Gonthier et al., 2003). In contrast, free caffeic acid is well adsorbed in the stomach and in the small intestine (Olthof et al., 2001; Rechner et al., 2001) and its intake is associated with a higher plasma concentration and urinary excretion of intact caffeic acid and its tissular metabolites (Gonthier et al., 2003).

Since the beginning of the 90s, the food industry has developed a number of innovative products containing health promoting components. Relevant examples are represented by selenium-enriched potatoes, fermented milk containing probiotics, milk enriched in ω3 fatty acids and dairy products containing phytosterols. Such products are known as functional foods, i.e. foods that are *"satisfactorily demonstrated to affect beneficially one or more target functions in the body, beyond adequate nutritional effects, in a way that is relevant to either improved state of health and well-being and*/*or reduction of risk of disease ",* as defined by the International Life Sciences Institute Europe, Diplock et al., 1999).

It has now been found that foods with a high content of caffeic acid in the free form can be prepared by fermenting a food matrix with a high content of antioxidant substances in the presence of a selected dairy probiotic bacterial starter.

### Description of the invention

The present invention relates to polyfunctional fermented food products containing high amounts of free caffeic acid, to a method for the preparation thereof and to probiotic bacteria for the preparation of the food product.

The food products according to the present invention are typically prepared by fermenting a food matrix with a high content of hydroxycinnamic acids, in particular chlorogenic acid, in the presence of microorganisms of the *Lactobacillus* species suitably selected for cinnamoyl esterase activity.

According to a preferred embodiment of the invention, the *Lactobacillus* selected for cinnamoyl esterase activity is a strain of *Lactobacillus helveticus, Lactobacillus acidophilus* or *Lactobacillus fermentum.*

Strains of *Lactobacillus helveticus* proved to be particularly suited for the purposes of the invention. One of said strain, isolated by means of the selection method detailed below, referred to hereinafter as *Lactobacillus helveticus* MIMLh5, will be used as an exemplary probiotic microrganism for use in the present invention.

Suitable strains may be selected by screening for the production of cinnamoyl esterase activity by thin layer chromatography (TLC) employing ethyl ferulate and chlorogenic acid and assaying the esterase activity of microorganisms obtained from public collections or isolated from natural sources such as human or animal organs, cheese starter cultures and the like. Microorganisms active on both substrates are preferably selected as the probiotic component of the food product of the invention. The selected microorganism will preferably be selected taking also into account other desirable properties such as the ability to survive the gastric transit, the stability towards bile, the adhesion properties to human intestinal cells. Test methods foe assaying said properties are well known.

The expression "food matrix" means a food mixture wherein at least one of the food product contains a high amount of hydroxycinnammic acids, particularly ferulic acid and chlorogenic acid. Examples of such foods are fruits, such as apples, pears, berries, red grapes, and juices obtained therefrom and beverages, such as tea, either black or green and coffee. The food matrix may also contain milk, preferably skimmed milk, to promote the growth of the microorganism, and optionally an antioxidant, preferably ascorbic acid, in order to prevent browning.

Particularly preferred is a food product obtained by fermenting a food mixture consisting of apple pulp and/or juice, milk and green tea. Among apples, the most preferred is the Renetta cultivar.

The fermented products of the invention contain an amount of free caffeic acid ranging from 100 to 250 mg/l; in particular, the fermented product obtained from Renetta apples and green tea contains about 185 mg/l free caffeic acid. Thus, for a 100 ml daily intake of fermented product of the invention, the amount of caffeic acid ingested could significantly increase the daily amount of phenolic compounds absorbed (Scalbert and Williamson, 2000; Scalbert et al., 2002) and have a significant biological effect on the consumer (Simonetti et al., 2001; Nardini et al., 2002; Chiang et al., 2003).

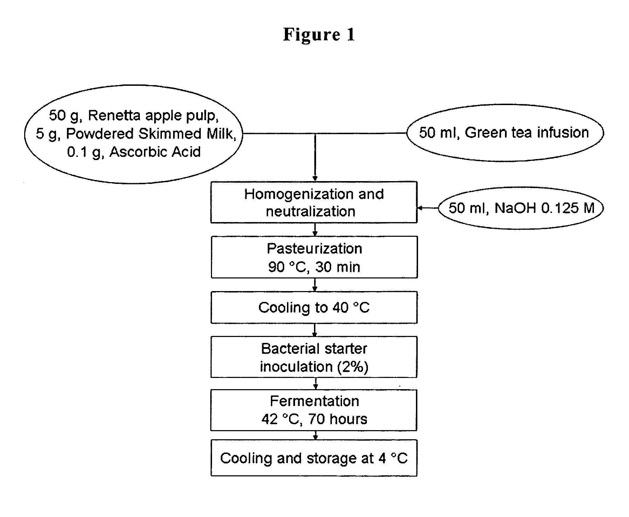

The product of the invention may be prepared by a process outlined in Figure 1.

Typically, the food matrix is first homogenized and neutralized, then submitted to pasteurization. After cooling, usually to about 40°C, the food matrix is inoculated with the microorganism having the desired cinnamoyl esterase activity and incubated for 60 - 80 hours, then cooled and stored at low temperature, usually at about 4°C.

Studies on rats and human volunteers have shown that, differently from chlorogenic acid, caffeic acid is efficiently adsorbed through the upper part of the gastrointestinal tract and is found in the body either intact or in glucuronidated, sulphated and *O*-methylated forms (ferulic and isoferulic acids), increasing the total antioxidant status of plasma (Olthof et al., 2001; Rechner et al., 2001; Nardini et al., 2002; Natella et al., 2002).

The total antioxidant effect of the food product of the invention was evaluated in comparison with other fermented dairy products according to a method known as FRAP *(Ferric Reducing Ability of Plasma),* already successfully applied on a variety of food products (Pellegrini et al., 2003); this method allows to determine the reduction of Fe³⁺ ions to Fe²⁺ ions in acidic conditions. The total antioxidant power of the product of the invention was much higher than that of the other fermented dairy products tested and also higher than beverages having a high concentration of antioxidant compounds, such as red wine and black tea.

The food of the invention is also endowed with remarkable probiotic properties, since it may contain up to about 10⁹ live cells/ml. The viability remains unaffected after three weeks storage at 4°C. This concentration is sufficient to provide a dose of 2 billion of viable bacterial cells/day, which promotes a probiotic effect (Sornikova et al., 1997a, 1997b). In particular, experiments in simulated gastric juices and bile solution showed a survival rate of *L. helveticus* MIMLh5 very similar to the control strain *Lactobacillus rhamnosus* GG (LGG), which is the most studied probiotic and is known to survive the gastrointestinal transit (Goldin et al., 1992). Adhesion experiments on Caco-2 cells were also performed in order to establish if the bacterial starter could adhere to human intestine, since adhesion of bacteria to human intestine is a prerequisite for even temporary colonization of the gastrointestinal tract. Therefore, adhesion is a desirable property for probiotic bacterial strains. The adhesion ability of *L. helveticus* MIMLh5 was significant and resulted very similar to the control strain LGG, which is known to be able to transiently colonize the human intestinal tract (Alander et al., 1999).

### DESCRIPTION OF THE FIGURES

Fig. 1. Flow sheet of the preparation of the fermented food product.
Fig. 2. HPCL chromatograms of the methanol extracts of a food product according to the invention before (A) and after (B) fermentation with *Lactobacillus helveticus* MIMLh5.
Fig. 3. Antioxidant activity of 10 different fermented food products as determined through FRAP assay. RAU = relative antioxidant units. PFFP = polyfunctional food product of the invention; ACE juice = commercial orange and carrot juice enriched in vitamins A, C and E; Actimel^{®} = probiotic fermented milk from Danone.
Fig. 4. Tolerance of *Lactobacillus helveticus* MIMLh5 and *Lactobacillus rhamnosus* GG to simulated gastric juices at pH 3 and 2 and to bile. A: Graphical representation of the viable bacterial cells before (T0) and after 3 hours (T3) of incubation under stress condition; B: bacterial cell counts (concentration of viable cells reported as CFU/ml).

The invention will be now illustrated in greater detail in the following experimental section.

### EXPERIMENTAL SECTION

### Materials and methods

### Bacterial strains and culture conditions

A total of 100 bacterial strains selected from 38 lactobacilli, 29 bifidobacteria, 25 carnobacteria, 6 enterococci and 2 streptococci (Table 1) were screened for cinnamoyl esterase activity. The lactobacilli were cultivated in MRS medium (Difco, Detroit, MI, USA) at 37°C, with the exception of *L. helveticus* that was incubated at 42°C. The bifidobacteria were grown in MRS medium supplemented with 0.5% cysteine hydrochloride, at 37°C and in strictly anaerobic conditions. The carnobacteria were cultivated in APT medium (Difco) at 30°C and the enterococci and streptococci were grown in M17 medium (Difco) at 37 and 42°C, respectively.

### Screening for cinnamoyl esterase activity

The bacterial strains were grown in 1 ml of liquid medium for 72 hours in the presence of 0.5 mg/ml of the reference substrate, ethyl ferulate or chlorogenic acid (Sigma, St. Louis, MO, USA). Hydroxycinnamic acids were extracted as follows: 0.25 ml ethyl acetate were added to the broth culture and vortexed for three minutes; after centrifugation, 0.2 ml of the organic phase were transferred into a new tube and the procedure was repeated.

Thin layer chromatography (TLC) was performed on silica gel 60 F254 plates (Merck, Milan, Italy) detected by treatment with a solution of 20 g of ammonium molibdate and 0.4 g of cerium sulfate in 400 ml of 10% aq. sulfuric acid followed by heating at 120°C.

For HPLC analyses, ethyl acetate was removed by evaporation under N₂ stream and the pellet was resuspended in 1 ml of methanol. Samples (50 µl) were injected onto a C18 reversed-phase analytical column (Hypersil ODS, 5 µm, 4.6 × 250 mm) previously equilibrated with 5% acetonitrile in methanol. Elution was performed at 1 ml min⁻¹ with solvents A, acetonitrile, and B, methanol, with the following gradient: from 5% to 95% B in 30 min, from 90% to 10% B in 10 min, back to 95% B in 5 min, 95% B for 5 min. Elution was monitored at 299 nm with a UV 655°-22 Merck-Hitachi D-2000 detector. Retention times: chlorogenic acid 9.8 min, caffeic acid 12.4 min.

### Preparation of the fermented food product

All the steps for the preparation of the food product are summarized in Fig. 1. All the ingredients were mixed to homogeneity with a traditional mixer: 50 g of apple cultivar Renetta pulp, 5 g of powdered skimmed milk (Difco), 0.1 g of ascorbic acid and 50 ml of green tea infusion (prepared leaving 2 g of dried Ceylon green tea leaves in 100 ml hot water for ten minutes). The pH was neutralized with 0.125 M NaOH and the food preparation was pasteurized at 90°C for 30 min. After cooling to around 40°C, the bacterial starter was inoculated (0.2 ml of an 18 hours broth culture into 10 ml of food preparation). Finally, the food product was incubated at 42°C for 70 hours. After fermentation, the food product was stored at 4°C.

### Extraction of hydroxycinnamates from the food product

The pH of the food product was brought to 1.5 with HCl. One gram of the acidified sample was mixed with 5 ml methanol and 0.1% ascorbic acid in a 50 ml Falcon tube. The suspension was incubated with stirring at 4°C for 1 hour and then centrifuged at 8000 rcf for 5 min at 4°C. The supernatant was transferred to a new tube and the pellet was resuspended in 5 ml methanol and treated as before.

The supernatants were stored at -20°C. HPLC analyses of the methanol extracts were performed as described above.

### Determination of the survival of the bacterial starter

The concentration of viable bacterial cells in the food product was calculated as follows. The bacterial cells were recovered by centrifugation and washed with sterile saline. Dead cells were stained with propidium iodide (Sigma) and counted through a fluorescence microscope. The data were reported as percentage of dead cells against the total number of cells counted in bright field microscope observation. Three different samples and twenty different microscope fields for each sample were counted. The total number of bacterial cells per ml of food sample was determined microscopically after staining with 4'-6-diamidino-2-phenylindole (DAPI count).

### Estimation of the antioxidant power of food products

The antioxidant activity of food products was determined with the FRAP method (Ferric Reducing Ability of Plasma, Benzie and Strani 1996; Pellegrini et al. 2003). Briefly, 30 µl of a 1:50 dilution of food product in methanol were added to 900 µl of TPTZ buffer (25 ml of 0.3 M acetate buffer pH 3.6, 2.5 ml of 5.41 g/l of FeCl₃ and 2.5 ml of 3.12 g/l 2,4,6-tripyridyl-1,3,5-triazine in 0.04 M HCl). The sample was incubated at 37°C for 10 min and immediately read spectrophotometrically at 595 nm. For each food sample four replications in at least two independent experiments were performed.

### Evaluation of the bacterial resistance to simulated gastric juices and bile

One hundred microliters of bacterial culture containing about 10⁸ to 10⁹ cfu/ml were transferred to 6 ml of each of the following buffers: simulated gastric juice at pH 2 containing 1 g/l peptone (Difco), 0.1 M KCl, 500 U/ml pepsin (Sigma); simulated gastric juice at pH 3 containing 0.125 M NaCl, 0.045 M NaHCO₃, 0.007 M KCl, 500 U/ml pepsin; bile solution containing 1 g/l peptone and 0.3% oxgall (Difco) in 0.1 M phosphate buffer at pH 6.5. The number of surviving cells was determined after 3 hours of anaerobic incubation at 37°C by plating on MRS agar medium. The plates were incubated under anaerobic conditions for 3 days.

### Bacterial adhesion to Caco-2 cell line

Caco-2 cells (American Type Culture Collection) were routinely grown in 6-wells microtiter plates on microscopy cover glasses in Dulbecco's Modified Eagle's Medium supplemented with 10% (v/v) heat-inactivated (30 min at 56°C) fetal calf serum, 100 U/ml penicillin, 100 mg/ml streptomycin, 0.1 mM non-essential amino acids, 2 mM L-glutamine (complete medium) and incubated at 37°C in a water-jacketed incubator under 5% CO₂. The culture medium was changed twice a week. For adhesion assay experiments, the cells were used 15 days after confluence (fully differentiated cells). Cell monolayers were carefully washed twice with phosphate-buffered saline pH 7.3 (PBS) before addition of bacterial cells. The bacterial cell concentration of an overnight culture was determined microscopically after DAPI staining. Approximately, 2 × 10⁸ cells for each strain were incubated with a monolayer of fully differentiated Caco-2 cells. After 1 h at 37°C under anaerobic conditions, all monolayers were washed 3 times with PBS to release the unbound bacteria. The cells were then fixed with 3 ml of methanol and incubated for 8 min at room temperature. After removal of the methanol, the cells were stained with 3 ml of Giemsa stain solution (1:20) (Carlo Erba, Milan, Italy) and left 30 min at room temperature. The wells were then washed until no colour was observed in the washing solution and dried in an incubator for 1 h. Microscopy cover glasses were then removed from the microtiter wells and examined microscopically (magnification x 100) under oil immersion. The adherent bacteria in 20 randomly selected microscopic fields were counted and averaged. All experiments were performed in duplicate.

### Results

### Selection of bacteria with cinnamoyl esterase activity

100 food or human intestinal bacterial strains were screened by thin layer chromatography (TLC) for the production of cinnamoyl esterase activity, employing ethyl ferulate and chlorogenic acid as reference substrates.

When ethyl ferulate was used, 24 out of the 100 tested strains displayed a detectable TLC band corresponding to ferulic acid (Table 2). With chlorogenic acid, significant hydrolysis of the substrate was observed by 17 out of the 24 strains positive with ethyl ferulate. All the other strains resulted negative. In particular, none of the bifidobacteria tested showed detectable cinnamoyl esterase activity. The strains among the 71 lactic acid bacteria that resulted undoubtedly positive on both substrates belonged to the species *Lactobacillus helveticus, Lactobacillus acidophilus* and *Lactobacillus fermentum* (Table 2).

The percentage of chlorogenic acid hydrolyzed by the 17 positive bacterial strains was determined by HPLC analysis. Two strains belonging to the *L. helveticus* species, named MIMLh5 and SIM7, apparently hydrolyzed all the chlorogenic acid used in the experiments (Table 2) and for this reason they were taken into consideration for the preparation of food products.

### Characterization of the fermented food product

*Lactobacillus helveticus* MIMLh5 showed better performances when grown in milk and apple juice than *L. helveticus* SIM7 (data not shown). Consequently, *L. helveticus* MIMLh5 was selected as starter for the production of the food product.

After fermentation, the food product had the texture of yoghurt and the smell typical of yoghurt and apple. The taste was fresh and the flavours of apple and green tea were dominant. The lactic fermentation by the microbial starter reduced the pH from about 7 to 3.6.

At the end of the fermentation process, the concentration of bacterial cells was 8.6 x 10⁸ cells/ml of food product. Experiments with propidium iodide showed that the decrease in the number of viable cells in food products after fermentation at 4°C was not significant after three weeks storage.

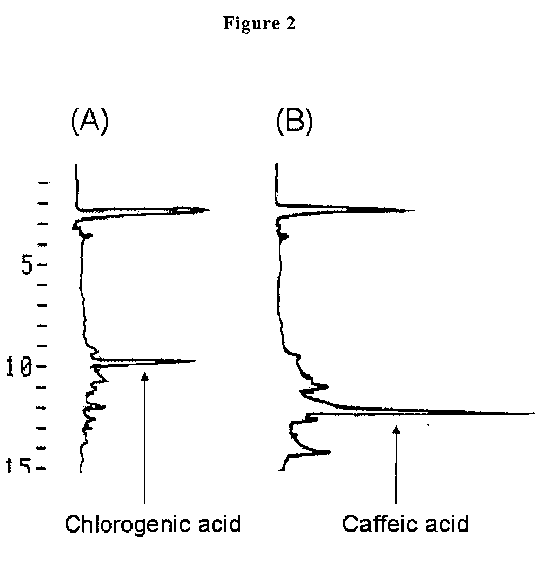

HPLC analyses confirmed that the bacterial starter converted chlorogenic acid to caffeic acid during the fermentation (Fig. 2). Before fermentation, the concentration of free caffeic acid was about 25 mg/l, while after fermentation the concentration was 185 mg/l.

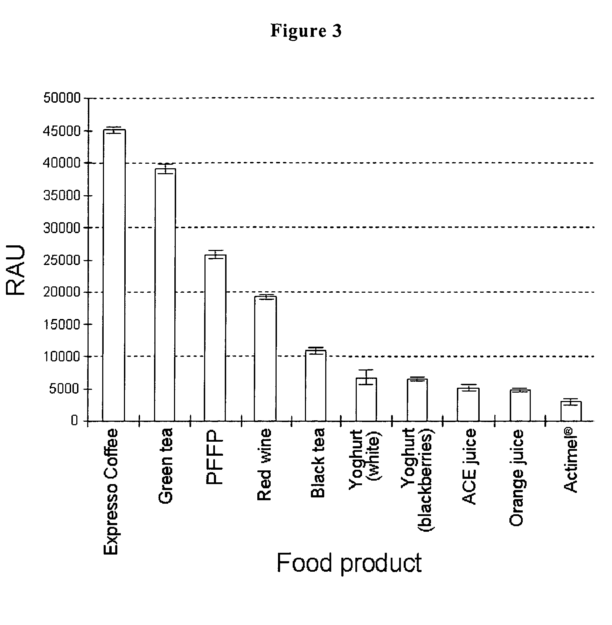

The antioxidant activity of the food product of the invention was determined and compared with several other commercial food products of the same type or with a high content of antioxidants. Only espresso coffee and green tea had an antioxidant power higher than the fermented food product of the invention; all the other products had a lower antioxidant power (Fig. 3). Particularly, red wine and black tea showed a 25 and 58% lower antioxidant activity, respectively (Fig. 3).

### Effect of simulated gastric juices and bile on the viability of Lactobacillus helveticus MIMLh5

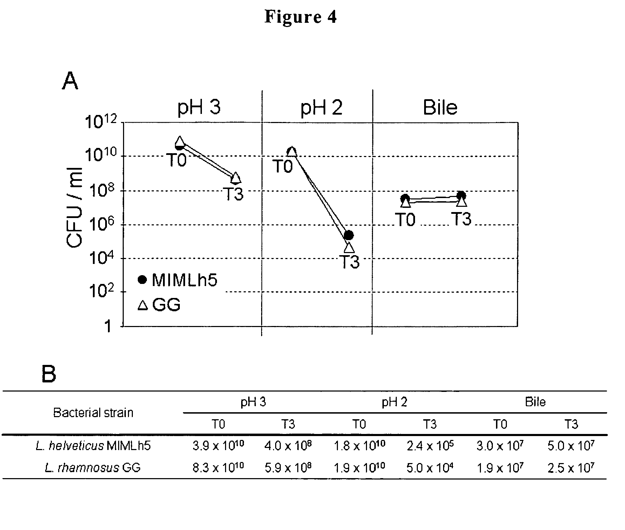

*Lactobacillus helveticus* MIMLh5 showed relatively high survival rate to simulated gastric juices at pH 2 and 3, comparable to the reference probiotic strain *Lactobacillus rhamnosus* GG (Fig. 4). 180-minutes exposure to pH 3.0 and pH 2.0 reduced viable counts of both bacterial strains by about 2 and 5 log cycles, respectively (Fig. 4B). Furthermore, oxgall concentrations of 0.3% did not affect the viability of the two strains during 3 hours of incubation at pH 6.5 under anaerobic conditions at 37°C (Fig. 4).

### Adhesion of Lactobacillus helveticus MIMLh5 to Caco-2 human colonic cell line

*Lactobacillus helveticus* LH5 displayed significant adhesion ability to Caco-2 cells. A significant proportion of cells remained attached to the Caco-2 monolayer after extensive washing, providing evidence that adhesion was not only the result of non-specific physical entrapment. Probiotic commercial strains *Lactobacillus rhamnosus* GG and *Bifidobacterium animalis* subsp. *lactis* BB12 were included as references in the experiments. Significant differences between the adhesion of *L. helveticus* LH5 and *L. rhamnosus* GG were not observed, while the adhesion ability of the other reference strain, *B. animalis* subsp. *lactis* BB12, resulted negligible under the conditions employed in the experiments.

**Table 1. Bacterial strains screened for cinnamoyl esterase activity.**

| Genus | Species | Number of strains (*) | Source |
|---|---|---|---|
| *Lactobacillus* | | 6 | Human gut |
| | *paracasei* | 4 | Fermented milk |
| | | 1 (DSM5622^{T}) | International culture collection |
| | *acidophilus* | 7 | Fermented milk |
| | | 2 (ATCC4356^{T} ) | International culture collection |
| | *helveticus* | 9 | Dairy starters |
| | *casei* | 4 | Human gut |
| | *fermentum* | 4 | Human gut |
| | *rhamnosus* | 1 (GG) | Human gut, probiotic commercial strain |
| *Carnobacterium* | *maltaromaticum* | 10 | Meat |
| | | 2 | Fish |
| | | 1 (DSM 20342^{T}) | Milk |
| | *divergens* | 12 (LMG9199^{T}) | Meat |
| *Enterococcus* | *faecalis* | 5 | Cheese |
| | *faecium* | 1 | Cheese |
| *Streptococcus* | *thermophilus* | 2 (DSM 20617^{T}) | Yoghurt |
| *Bifidobacterium* | *bifidum* | 7 | Human faeces |
| | *pseudocatenulatum* | 7 | Human faeces |
| | *longum* | 7 | Human faeces |
| | *animalis* subsp. *lactis* | 4 (BB12) | probiotic commercial products |
| | *adolescentis* | 4 | Human faeces |
| | | TOT. = 100 | |

**Table 2. Specific activities of the 24 bacterial strains resulted positive for the presence of cinnamoyl esterases. Levels of activity: - = not detectable; +/- = very weak/uncertain; + = weak; ++ = good; +++ = strong. NT = not tested. Eth. Fer. = ethyl ferulate; Chl. acid = chlorogenic acid.**

| Genus | Species | Strain | TLC | | HPLC (Chl. acid) |
|---|---|---|---|---|---|
| | | | Eth. Fer | Chl. acid | |
| | *helveticus* | MIMLh5 | +++ | +++ | 100% |
| | | SIM7 | +++ | +++ | 100% |
| | | CBT17 | ++ | +++ | 92.5% |
| | | LH22 | +++ | +++ | 91.5% |
| | | LH28 | ++ | ++ | 70% |
| | | LH23 | ++ | + | < 5% |
| | | LH4 | ++ | +/- | < 5 % |
| | | LH164 | ++ | - | NT |
| | *acidophilus* | ATCC4356 ^{T} | +++ | ++ | 81.4% |
| *Lactobacillus* | | LA47 | ++ | ++ | 70.5% |
| | | LA48 | +++ | ++ | 59.4% |
| | | LA51 | +++ | ++ | 56.6% |
| | | LA53 | ++ | ++ | 54.8% |
| | | LA46 | +++ | +/- | <5% |
| | | LA50 | ++ | - | NT |
| | *fermentum* | LB12, LB18 | ++ | +/- | < 5 % |
| | | LB19 | ++ | + | 41.6% |
| | *casei* | LB21, LB22 | + | - | NT |
| | *rhamnosus* | GG | + | +/- | < 5% |
| *Enterococcus* | *faecalis* | SMT, TD1, TA15 | + | - | NT |

### Bibliography

Aihara K. et al., J Am Coll Nutr. 2005;24:257-65.
Alander M. et al., Appl Environ Microbiol. 1999;65:351-4.
Benzie IF. et al., Anal Biochem. 1996;239:70-6.
Boyer J. et al., Nutr J. 2004;12:3-5.
Chauviere G,. J Gen Microbiol. 1992; 138:1689-96.
Chiang LC et al., Planta Med. 2003;69:600-4.
Clifford MN, J Sci Food Agric. 1999;79:362-72.
Diplock AT et al., Br J Nutr. 1999; 81 (Suppl 1): S1-S28.
Foley S. et al., Free Radic Biol Med. 1999;26:1202-8.
Goldin BR. et al., Dig Dis Sci. 1992;37:121-8.
Gonthier M. et al., J Nutr. 2003;133:1853-59.
Greene JD. et al., Appl Environ Microbiol. 1994;60:4487-94.
Herrmann K., J Food Technol. 1976; 11:433-8.
Johnson-Henry KC. et al. Cell Microbiol. 2006; Epub ahead of print.
Kasai H. et al., Food Chem Toxicol. 2000:38:467-71.
Kono Y. et al., Biochem J. 1995;312:947-53.
Kuhnau J., World Rev Nutr Diet. 1976;24:117-91.
Lafay S. et al., Int J Vitam Nutr Res. 2005;75:119-25.
Lafay S. et al., J Nutr. 2006:136:1192-7.
Leblanc J. et al., Clin Diagn Lab Immunol. 2004; 11:1171-81
Mori H. et al., Cancer Lett. 1986;30:49-54.
Nardini M. et al., Free Radic Biol Med. 1995; 19:541-52.
Nardini M. et al., J Agric Food Chem. 2002;50:5735-41.
Natella F. et al., J Agric Food Chem. 2002:50:6211-6.
Olthof MR. et al., J Nutr. 2001;131:66-71.
Pellegrini N. et al., J Nutr. 2003;133:2812-9.
Rababah TM. et al., J Agric Food Chem. 2004;52:5183-6.
Rechner AR. et al., Free Radic Biol Med. 2001;30:1213-22.
Rice-Evans CA. et al., Free Radic Biol Med. 1996;20:933-56.
Scalbert A. et al., J Nutr. 2000; 130(8S Suppl):2073S-85S.
Scalbert A. et al., Biomed Pharmacother. 2002;56:276-82.
Shibata H. et al., Biosci Biotechnol. Biochem. 1999;63:1295-7.
Shornikova AV. et al., J Pediatr Gastroenterol Nutr. 1997a;24:399-404.
Shornikova AV. et al., Acta Paediatr. 1997b;86:460-5.
Simonetti P. et al., J Agric Food Chem. 2001;49:5964-8.
Spencer JP. et al., FEBS Lett. 1999;458:224-30.
Suzuki A. et al., Hypertens Res. 2002;25(1):99-107.
Takeda H et al., Eur J Pharmacol. 2002;449:261-7.
Tanaka T. et al., Carcinogenesis. 1993; 14:1321-25.
Tanaka T. et al., Basic Life Sci. 1990;52:429-40.
Tsuchiya T. et al., Biosci Biotechnol Biochem. 1996;60:765-8.
Vrhovsek U. et al., J Agric Food Chem. 2004;52:6532-8.
Zhou J. et al., J Clin Biochem Nutr. 1993;15:119-25.

## Claims

1. A fermented food product comprising a food matrix with a high content of hydroxycinnamic acid derivatives and a microorganism having cinnamoyl esterase activity.

2. A food product according to claim 1 wherein the microorganism is a *Lactobacillus.*

3. A food product according to claim 2 wherein the microorganism is selected from a strain of *Lactobacillus helveticus, Lactobacillus acidophilus* and *Lactobacillus fermentum.*

4. A food product according to claim 3 wherein the microorganism is a *Lactobacillus helveticus* strain.

5. A food product according to any one of claims 1 to 4 wherein the food matrix derives from food having an high content of chlorogenic acid.

6. A food product according to claim 5 wherein said food having an high content of chlorogenic acid is derived from apples, pears, berries, teas and coffee.

7. A food product according to any one of claims from 1 to 6 further comprising milk and/or an antioxidant.

8. A food product according to claim 5 or 6 wherein the food matrix comprises apple pulp and/or juice, milk, green tea and ascorbic acid.

9. A food product according to claim 5 wherein the apple pulp and/or juice is obtained from *Renetta* apples.
